# EUROPÄISCHE PATENTSCHRIFT

(11) **EP 0 639 161 B1**
(45) Veröffentlichungstag und Bekanntmachung des Hinweises auf die Patenterteilung: **14.08.1996**
(21) Anmeldenummer: 93911743.8
(22) Anmeldetag: 05.05.1993
(51) Int. Cl.: C02F 3/12, C02F 3/28, C12M 1/40

(54) **WIRBELSCHICHTFERMENTER**
FLUIDIZED-BED FERMENTER
FERMENTATEUR EN LIT FLUIDISE

(30) Priorität: 07.05.1992 DE 4214896
(43) Veröffentlichungstag der Anmeldung: 22.02.1995
(73) Patentinhaber: SCHERING AKTIENGESELLSCHAFT, D-13342 Berlin (DE)
(72) Erfinder: DONNER, Christoph, D-1532 Kleinmachnow (DE); SOKOLOWSKY, Stephan, D-1000 Berlin 33 (DE); REINKE, Lothar, D-1000 Berlin 12 (DE)
(86) Internationale Anmeldenummer: DE9300414
(87) Internationale Veröffentlichungsnummer: WO9322246

(56) Entgegenhaltungen:
- EP-A- 0 433 139
- BE-A- 900 159
- DE-A- 4 011 649
- FR-A- 2 102 216

## Beschreibung

Die Erfindung betrifft einen Wirbelschichtfermenter, welcher gekennzeichnet ist durch einen auf der Spitze stehenden kegelförmigen Fermentationsraum (1) mit zwei gegebenenfalls mit Steuerventilen (4 und 5) versehenen Zuführungsleitungen (2 und 3) von denen die eine (2) vertikal ausgerichtet in der Kegelspitze und die zweite (3) nicht vertikal ausgerichtet im Kegelmantel in der 0,02 bis 0,3-fachen Fermentationsraumhöhe angeordnet ist und einen oberhalb des Fermentationsraums (1) befindlichen Sedimentationsraum (6), welcher ein oder zwei gegebenenfalls mit Steuerventilen (9 und 10) versehene Abflußleitungen (7 und 8) besitzt.

Die besondere Formgebung des erfindungsgemäßen Wirbelschichtfermenters bewirkt, daß in dessen Fermentationsraum (1) eine turbulente Strömung herrscht, wenn man die zu fermentierende Flüssigkeit gleichzeitig über die vertikale und horizontale Zuführungsleitung (2 und 3) einspeist. Diese Strömung geht dann im Sedimentationsraum (6) in eine quasi laminare Strömung über. Wird ein derartig kontinuierlich beschickter Fermenter mit einer Mikroorganismen-Kultur beimpft so bildet diese nach einer Anwachsphase infolge der turbulenten Strömung pelletartige Agglomerate aus, die im Fluidatbett des Fermentationsraums (1) schweben und im Sedimentationsraum (6) wieder absinken. Infolge dieser Gegebenheiten eignet sich der erfindungsgemäße Wirbelschichtfermenter wesentlich besser zur kontinuierlichen Fermentation großer Flüssigkeitsmengen über einen langen Zeitraum hin als dies bei vorbekannten Wirbelschichtfermentern, wie beispielsweise der in der Europäischen Patentschrift EP-B-0258611 beschriebenen Vorrichtung, der Fall ist. Demzufolge ist der erfindungsgemäße Wirbelschichtfermenter beispielsweise zur biologischen Abwasser- oder Grundwasseraufbereitung besonders geeignet.

Die Formgebung des erfindungsgemäßen Wirbelschichtfermenters ist im wesentlichen durch die Art der Mikroorganismen-Kulturen bestimmt, die in ihm verwendet werden sollen. Sind dies Mikroorganismen-Kulturen, die relativ rasch sedimentieren, wie zum Beispiel Mikroorganismen-Immobilisate, wie sie in der internationalen Patentanmeldung WO 88/08825 beschrieben sind oder Pilzkulturen, so wird man vorzugsweise Wirbelschichtfermenter mit einem Fermentationsraum (1) und Sedimentationsraum (6) von relativ geringer Höhe verwenden. Bei relativ schlecht sedimentierenden Mikroorganismen-Kulturen, wie dies in der Regel Bakterienkulturen sind, wird man vorzugsweise Wirbelschichtfermenter mit relativ hohem Fermentationsraum (1) und Sedimentationsraum (6) bevorzugen. Es ist aber nicht erforderlich, daß für jede verwendete MikroorganismenKultur ein speziell ausgestalteter Fermenter angewendet wird; durch eine entsprechende Regulierung der Zuflußgeschwindigkeiten der zu fermentierenden Flüssigkeiten durch die vertikale und nicht vertikale Zuführungsleitung (2 und 3) läßt sich in der Regel auch bei für den speziellen Fall nicht optimal ausgestalteten erfindungsgemäßen Wirbelschichtfermentern eine kontinuierliche Fermentation realisieren.

In der Regel wird der erfindungsgemäße Wirbelschichtfermenter so ausgestaltet sein, daß der obere Durchmesser des Fermentationsraumes (1) 0,1 bis 0,8 mal so groß ist wie dessen Höhe. Bei diesen Zahlenwerten ist nicht berücksichtigt, daß der Fementationsraum in aller Regel eine abgestumpfte Kegelspitze besitzt, was schon aus dem Grunde zweckmäßig ist, damit der Fermenter leichter gereinigt werden kann.

Es wurde bereits erwähnt, daß die nicht vertikal ausgerichtete Zuführungsleitung (3) im Kegelmantel in der 0,02 bis 0,3 - insbesondere 0.05 bis 0,15-fachen - Fermentationsraumhöhe angeordnet ist,. wobei beide Zuführungsleitungen so ausgerichtet sind, daß bei der Inbetriebnahme des Fermenters im Fermentationsraum ein Wirbelschichtbett erzeugt wird. Die nicht vertikal ausgerichtete Zuführungsleitung (3) kann horizonal so angeordnet werden,daß die von der Eintrittsöffnung dieser Zuleitung zur Fermentationsraumesachse weisende horizontale Gerade diese Achse kreuzt. Zur Erzeugung einer gut reproduzierbaren turbulenten Strömung ist es aber zweckmäßig, daß die Längstachse der nicht vertikal ausgerichteten Zuleitung und die von der Eintrittsöffnung dieser Zuleitung zur Fermentationsrumachse weisenden horizontalen Geraden in der Vertikalen und/oder Horizonalen einen Winkel bilden, der in der Vertikalen den Wert 70° und in der Horizontalen den Wert 60° nicht übersteigt. Ist diese Zuführungsleitung (3) vertikal nach oben gerichtet angeordnet, so beträgt der Winkel vorzugsweise 10° bis 60 ° (insbesondere 30 ° bis 40 °), wenn die Leitung vertikal, aber nach unten gerichtet angeordnet ist, hat der Winkel zweckmäßigerweise einen Wert zwischen 10 ° und 60 °. (insbesondere 20 ° bis 45 °) Falls die Zuführungsleitung (3) gegebenenfalls zusätzlich horizontal versetzt angeordnet ist, beträgt der Winkel vorzugsweise 5° bis 60° und insbesondere 20° bis 45°

Es ist für den Fachmann offenkundig, daß die Begriffe "horizontal" und "vertikal" nicht im mathematischen Sinne zu verstehen sind, sondern, daß die Zuführungsleitungen innerhalb der üblichen Toleranzen von einer exakten horizontalen oder vertikalen Anordnung abweichen können. In der Regel werden die Zuführungsleitungen (2 und 3) mit den üblichen Steuerventilen (4 und 5) versehen und über eine gemeinsame Leitung mit einem zur Aufnahme der zu fermentierenden Lösung bestimmten Vorratsgefäß verbunden sein.

Es wurde bereits erwähnt, daß der erfindungsgemäße Wirbelschichtfermenter einen oberhalb des Fermentationsraumes (1) befindlichen Sedimentationsraum (6) hat, der ein oder vorzugsweise zwei gegebenenfalls mit Steuerventilen (9 und 10) versehene Abflußleitungen (7 und 8) besitzt.

Dieser Sedimentationsraum (6) kann so gestaltet sein, daß er eine übergangslose Fortsetzung des kegelförmigen Fermentationsraums darstellt; andererseits kann er beispielsweise auch so ausgestaltet sein, daß er aus ein oder zwei gegebenenfalls mit einer konischen Erweiterung versehenen, zylindrischen Bauteilen besteht. In diesem Falle kann er so dimensioniert sein, daß sein oberer Druchmesser das 1 bis 3-fache (insbesondere das 1,5 bis 2,5 Fache) des oberen Durchmessers des Fermentationsraumes (1) beträgt. Der Sedimentationsraum (6) ist vorzugsweise so dimensioniert, daß er die 0,2 bis 0,5-fache Höhe des Fermentationsraumes (1) besitzt. Der Sedimentationsraum (6) besitzt ein oder vorzugsweise zwei gegebenenfalls mit Steuerventilen (9 und 10) versehene Abflußleitungen. Zwei Abflußleitungen sind dann zweckmäßig, wenn beabsichtigt ist, einen Teil der fermentierten Flüssigkeit in den Fermentationskreislauf zurück zu führen um die zu fermentierende Flüssigkeit soweit zu verdünnen, daß eine praktisch vollständige Umwandlung der darin enthaltenen Substrate erreicht werden kann. In diesem Falle ist die der Rückführung dienende Abflußleitung zweckmäßigerweise etwas tiefer angeordnet (vorzugsweise 20 bis 40 % unterhalb der oberen Abflußleitung), als die dem Abfluß dienende Leitung.

Ebenso wie konventionelle Fermenter kann auch der erfindungsgemäße Wirbelschichtfermenter mit den üblichen Hilfsmitteln versehen werden, die eine Temperierung, pH-Wert-Steuerung, Belüftung und/oder Sterilisation des Fermenterinhaltes ermöglichen. Er kann wie auch konventionelle Fermenter aus Glas und/oder korrosionsfestem Metall gefertigt sein.

Anhand von Zeichnungen soll die Erfindung näher erläutert werden.

Es zeigen:
Figur 1 einen Längsschnitt durch einen erfindungsgemäßen Wirbelschichtfermenter.
Figur 2 einen Querschnitt durch den Wirbelschichtfermenter der Firgur 1 in Höhe der Ebene A-B.
Figur 3 einen Längsschnitt durch einen Wirbelschichtfermenter mit eingebauter Temperier-, Belüfiungs- und Sterilisiervorrichtung.
Figur 4 eine schematische Darstellung einer Fermentationsanlage mit einem erfindungs-gemäßen Wirbelschichtfermenter und
Figur 5 eine schematische Darstellung einer Fermentationsanlage mit zwei in Reihe geschalteten Wirbelschichtfermentern.

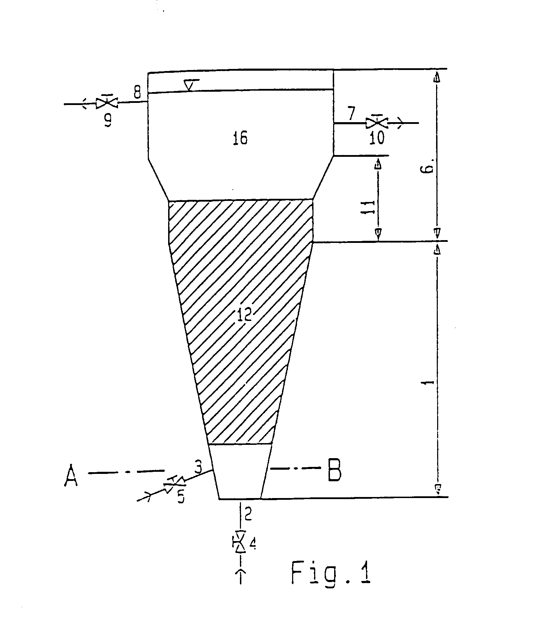

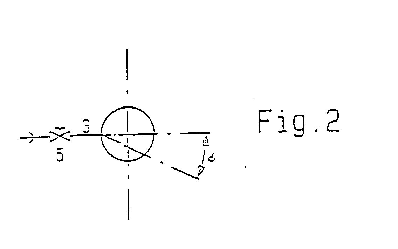

Der in Figur 1 dargestellte Wirbelschichtfermenter ist zur Abwasseraufbereitung mit Bakterien-Kulturen bestimmt. Er besteht aus dem kegelförmigen Fermentationsraum (1) mit abgestumpfter Kegelspitze dem Sedimentationsraum (6) sowie den mit Steuerventilen (4 und 5) versehenen Zuführungsleitungen (2 und 3) und den mit Steuerventilen (9 und 10) versehenen Abflußleitungen (7 und 8). Der Fermentationsraum (1) hat eine Höhe von 2300 mm und einen oberen Durchmesser von 600 mm. In ihm führt die vertikale Zuleitung (2) und in einer Höhe von 230 mm die nicht horizontale Zuleitung (3), die vertikal nach oben geneigt ist und mit der und mit der Horizontalen einen Winkel von 35° bildet. Am oberen Rand des Fermentationsraumes (1) ist der Sedimentationsraum (6) angeordnet. Dieser besteht aus zwei, mit einer konischen Erweiterung versehenen, zylindrischen Bauteilen, ist oben verschlossen und hat einen oberen Durchmesser von 1300 mm und eine Gesamthöhe von 1300 mm.(Die schraffierte Fläche 12 der Figur 1 soll den Bereich symbolisieren, in welchem die Bakterienkultur verwirbelt wird. Der mit der Ziffer 16 gekennzeichnete Raum soll den weitgehend bakterienfreien Raum symbolisieren). Unterhalb der oberen Abdeckung sind einander gegenüberstehend zwei Abflußleitungen (7 und 8) angebracht, von denen die eine, dem Ablauf der Fermentationsbrühe dienende Leitung (8) 250 mm und die der Rückführung dienende Leitung (7) 600 mm unterhalb der oberen Abdeckung des Sedimentationsraums angeordnet ist. Der Fermenter ist aus rostfreiem Stahl angefertigt. Aus Fig 2 ist erkennbar, daß die nicht vertikale Zuführungsleitung (3) mit der von ihrer Eintrittsöffnung zur Fermentationsachse weisenden Geraden einen Winkel a von 35 ° bildet.

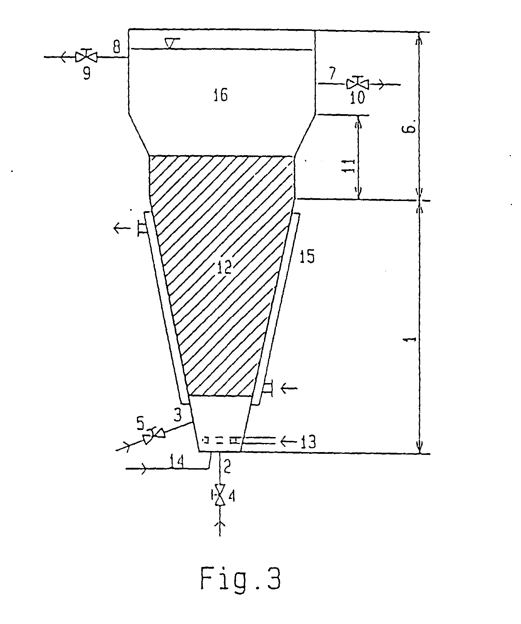

Der in Figur 3 dargestellte Wirbelschichtfermenter hat den gleichen Aufbau wie der in Figur 1 beschriebene, er hat aber zusätzlich noch eine dicht über dem Boden befindliche ringförmige mit Düsen versehene Zuluftleitung (13), eine Heißdampfleitung (14), die dazu dienen kann, den Fermenterinhalt zu sterilisieren und einen den Fermentationsraum umhüllenden Doppelmantel (15), zur Aufnahme der Temperierflüssigkeit.

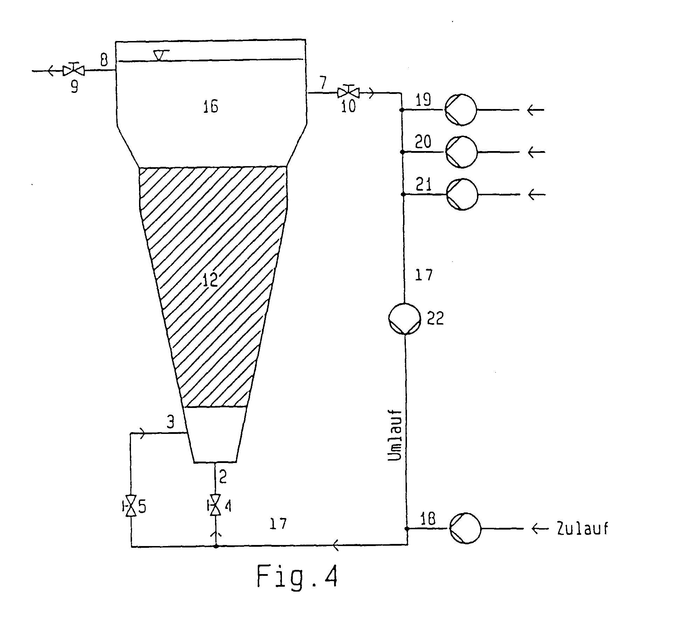

Die in Figur 4 schematisch dargestellte Fermentationsanlage besteht im Wesentlichen aus einem Wirbelschichtfermenter, wie in Figur 1 erläutert und einer mit einer Dosierpumpe (22) sowie einer Zuleitung zur Einspeisung von zur p_{H}-Wert steuernder Hilfsmittel (19), einer Zuleitung zur Einspeisung von H₂O₂ als Sauerstoffdonator (20), einer Zuleitung zur Einspeisung von Nährmedium (21) und einer Zuleitung zur Einspeisung von Abwasser (18), versehenen Rückführungsleitung (17), welche über eine Verzweigung mit den Zuführungsleitungen (2 und 3) verbunden ist.

Über die Zuleitung (18) werden stündlich 1 m³ Abwasser und der mit Nährmedium angereicherte auf p_{H} = 7 eingestellte Rücklauf in den mit einer aus dem entsprechenden Abwasserschlamm isolierten Bakterien-Mischkultur beimpften Fermenter eingespeist, wobei die Steuerventile (4 und 5) der Zuführungsleitungen (2 und 3) so eingestellt werden, daß im Fermenter nach Abschluß der Anwachsphase (ca.2 bis 6 Wochen) ein stabiles Fluidatbett mit konstanter Mikroorganismen-Dichte entsteht. Dann werden dem Fermenter über die eine Abflußleitung (7) stündlich 1 m³ gereinigtes Abwasser entnommen während über die andere Abflußleitung (8) pro Stunde 7 bis 14 m³ gereinigtes Abwasser in den Kreislauf zurückgeführt werden um das eingespeiste Abwasser oder Grundwasser zu verdünnen.

Mit dieser Fermentationsanlage ist es möglich, kontinuierlich pro Stunde 1 m³ Abwasser, welches mit Tetrahydrofuran, Diethylether und Diisopropylether verunreingt ist, über mehrere Monate hin so aufzureinigen, daß es maximal noch 5 % der genannten Verunreinigungen enthielt.

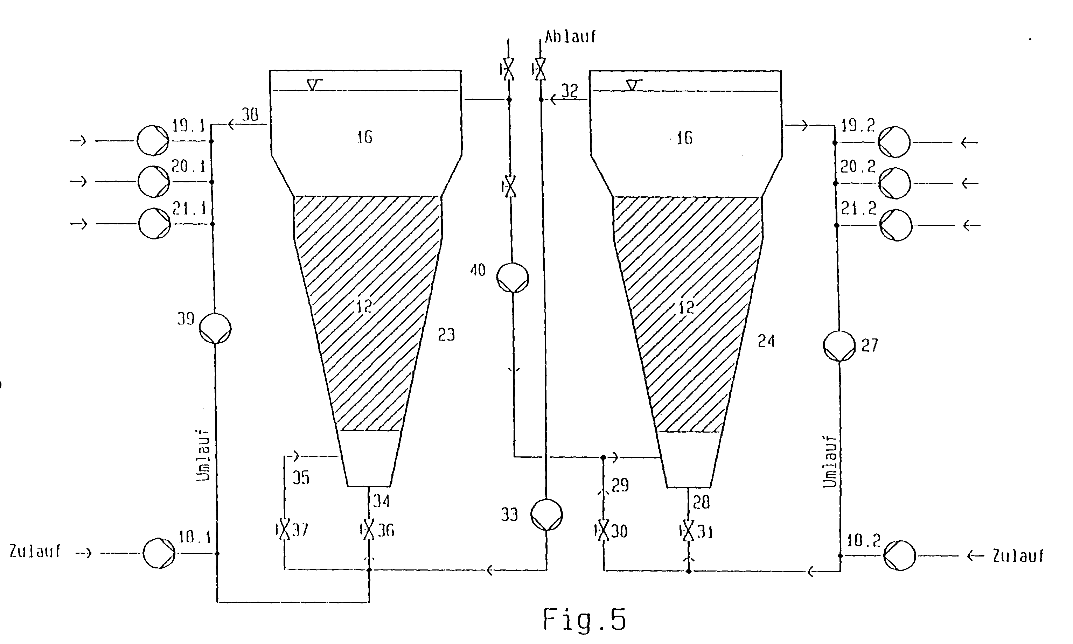

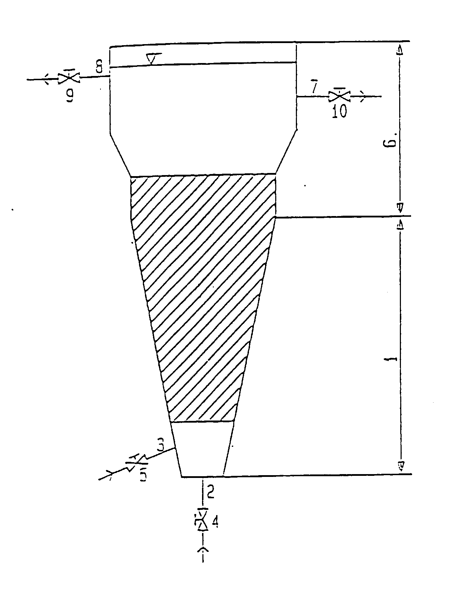

Letztlich sei die in Figur 5 schematisch dargestellte Fermentationsanlage erwähnt, die sich im Wesentlichen von der in Figur 4 skizzierten Vorrichtung dadurch unterscheidet, daß sie zwei Fermentationseinheiten (23 und 24) besitzt, die prinzipiell den gleichen Aufbau besitzen,wie der in Fig 4 dargestellte Einheit, wobei die von der Einheit 24 abführende Abflüßleitung 32 zur Einspeisung des Abwassers oder Grundwassers in die Fermentationseinheit 23 dient. Diese Anlage kann durch entsprechende Einstellung der Steuerventile so eingestellt werden, daß die Fermenter parallel oder in Reihe geschaltet sind.

Für den Fachmann ist offensichtlich, daß man die erfindungsgemäßen Wirbelschichtfermenter nicht nur zur Abwasser- oder Grundwasseraufbereitung einsetzen kann. Mögliche Anwendungsgebiete dieser Fermenter ergeben sich beispielsweise auch bei der kontinuierlichen fermentativen Herstellung wasserlöslicher Alkohole, Säuren oder Kohlehydrate, wie zum Beispiel Ethanol, Essigsäure, Zitronensäure, Galactose, L-Sorbose, die mikrobielle Synthese von Aminosäuren, wie Glutaminsäure, die Antibiotikaproduktion, oder die mikrobiologische Transformation von Steroiden.

## Patentansprüche

1. Wirbelschichtfermenter gekennzeichnet durch einen auf der Spitze stehenden kegelförmigen Fermentationsraum (1) mit zwei gegebenenfalls mit Steuerventilen (4 und 5) versehenen Zuführungsleitungen (2 und 3) von denen die eine (2) vertikal abgerichtet in der Kegelspitze und die zweite (3),nicht vertikal ausgerichtet im Kegelmantel in der 0,02 bis 0,3-fachen Fermentationsraumhöhe angeordnet ist und einen oberhalb des Fermentationsraums (1) befindlichen Sedimentationsraum (6), welcher ein oder zwei gegebenenfalls mit Steuerventilen (9 und 10) versehene Abflußleitungen (7 und 8) besitzt.

2. Wirbelschichtfermenter gemäß Patentanspruch 1, dadurch gekennzeichnet, daß der obere Durchmesser des Fermentationsraums (1) 0,1 bis 0,8 mal so groß ist wie dessen Höhe.

3. Wirbelschichtfermenter gemäß Patentanspruch 1 und 2, dadurch gekennzeichnet, daß der Fermentationsraum (1) eine abgestumpfte Kegelspitze besitzt.

4. Wirbelschichtfermenter gemäß Patentanspruch 1 bis 3, dadurch gekennzeichnet, daß die vertikal ausgerichtete Zuführungsleitung (2) und die nicht vertikal ausgerichtete Zuführungsleitung (3) so angeordnet sind, daß bei der Inbetriebnahme des Fermenters im Fermentationsraum ein Wirbelschichtbett erzeugt wird.

5. Wirbelschichtfermenter gemäß Patentanspruch 4, dadurch gekennzeichnet, daß der Winkel zwischen der Längsachse der nicht vertikal ausgerichteten Zuleitung (3) und der von der Eintrittsöffnung dieser Zuleitung zur Fermentationsraumachse weisenden horizontalen Geraden in der Vertikalen 70° und in der Horizontalen 60° nicht überschreitet.

6. Wirbelschichtfermenter gemäß Patentanspruch 1 bis 5, dadurch gekennzeichnet, daß der Sedimentationsraum (6) aus ein oder zwei, gegebenenfalls mit einer konischen Erweiterung versehenen, zylindrischen Bauteilen besteht.

7. Wirbelschichtfermenter gemäß Patentanspruch 6, dadurch gekennzeichnet, daß der obere Durchmesser des Sedimentationsraums (6) das 1 bis 3-fache des oberen Durchmessers des Fermentationsraumes (1) beträgt.

8. Wirbelschichtfermenter gemäß Patentanspruch 6 und 7, dadurch gekennzeichnet, daß der Sedimentationsraum (6) die 0,2 bis 0,5-fache Höhe des Fermentationsraumes (1) besitzt.

9. Verwendung von Wirbelschichtfermentern gemäß Patentanspruch 1 bis 8 zur Abwasser- oder Grundwasseraufbereitung.

## Claims

1. Fluidised bed fermenter characterised by a conical fermentation zone (1) standing on its vertex and having two supply pipes (2 and 3) that are optionally provided with control valves (4 and 5), one of the supply pipes (2) being vertically aligned in the vertex of the cone and the second (3) being non-vertically aligned in the casing of the cone at from 0.02 to 0.3 times the height of the fermentation zone, and characterised by a sedimentation zone (6) located above the fermentation zone (1), which sedimentation zone (6) has one or two discharge pipes (7 and 8) that are optionally provided with control valves (9 and 10).

2. Fluidised bed fermenter according to patent claim 1, characterised in that the upper diameter of the fermentation zone (1) is from 0.1 to 0.8 times as large as its height.

3. Fluidised bed fermenter according to patent claims 1 and 2, characterised in that the fermentation zone (1) has a truncated cone vertex.

4. Fluidised bed fermenter according to patent claims 1 to 3, characterised in that the vertically aligned supply pipe (2) and the non-vertically aligned supply pipe (3) are so arranged that when the fermenter is put into operation a fluidised bed is produced in the fermentation zone.

5. Fluidised bed fermenter according to patent claim 4, characterised in that the angle between the longitudinal axis of the non-vertically aligned supply pipe (3) and the horizontal straight line extending from the inlet opening of that supply pipe to the fermentation zone axis does not exceed 70° in the vertical direction and 60° in the horizontal direction.

6. Fluidised bed fermenter according to patent claims 1 to 5, characterised in that the sedimentation zone (6) consists of one or two cylindrical components optionally provided with a conical widening portion.

7. Fluidised bed fermenter according to patent claim 6, characterised in that the upper diameter of the sedimentation zone (6) is from 1 to 3 times the upper diameter of the fermentation zone (1).

8. Fluidised bed fermenter according to patent claims 6 and 7, characterised in that the sedimentation zone (6) is from 0.2 to 0.5 times the height of the fermentation zone (1).

9. Use of fluidised bed fermenters according to patent claims 1 to 8 for the treatment of effluent or ground water.

## Revendications

1. Fermenteur à lit fluidisé, caractérisé en ce qu'il est constitué d'un compartiment de fermentation (1) conique reposant sur le sommet, est équipé de deux conduites d'alimentation (2 et 3) éventuellement munies de vannes de régulation (4 et 5) dont l'une des conduites (2) est disposée verticalement au sommet du cône et la deuxième (3) est disposée non-verticalement dans la chemise du cône à une hauteur de 0,02 à 0,3 fois la hauteur du compartiment de fermentation et un compartiment de sédimentation (6) se trouvant au-dessus du compartiment de fermentation (1), lequel compartiment de sédimentation (6) est muni de conduites d'évacuation (7 et 8), éventuellement munies de vannes de régulation (9 et 10).

2. Fermenteur à lit fluidisé selon la revendication 1, caractérisé en ce que le diamètre supérieur du compartiment de fermentation (1) est de 0,1 à 0,8 fois la hauteur de celui-ci.

3. Fermenteur à lit fluidisé selon les revendications 1 et 2, caractérisé en ce que le compartiment de fermentation (1) a un sommet de cône tronqué.

4. Fermenteur à lit fluidisé selon les revendications 1 à 3, caractérisé en ce que la conduite d'alimentation (2) disposée verticalement et la conduite d'alimentation (3) disposée non verticalement sont disposées de telle façon qu'au moment de la mise en route du fermenteur il se forme un lit fluidisé dans le compartiment de fermentation.

5. Fermenteur à lit fluidisé selon la revendication 4, caractérisé en ce que l'angle formé par l'axe longitudinal de la conduite (3) disposée non verticalement et les droites horizontales qui pointent vers l'axe du compartiment de fermentation, devantl'orifice d'entrée de ces conduites ne dépasse pas dans la position verticale 70° et dans la position horizontale 60°.

6. Fermenteur à lit fluidisé selon les revendications 1 à 5, caractérisé en ce que le compartiment de sédimentation (6) est constitué d'un ou de deux éléments cylindriques munis éventuellement d'un élargissement conique.

7. Fermenteur à lit fluidisé selon la revendication 6, caractérisé en ce que le diamètre supérieur du compartiment de sédimentation (6) est une à trois fois le diamètre supérieur du compartiment de sédimentation (1).

8. Fermenteur à lit fluidisé selon les revendications 6 et 7, caractérisé en ce que le compartiment de sédimentation (6) est 0,2 à 0,5 fois la hauteur du compartiment de fermentation (1).

9. Utilisation de fermenteurs à lit fluidisé selon les revendications 1 à 8 pour le traitement des eaux de rejet ou des eaux souterraines.
